Europäisches Patentamt

European Patent Office

Office européen des brevets

(19)

(11) Veröffentlichungsnummer: **0 232 277**
**B1**

(12) **EUROPÄISCHE PATENTSCHRIFT**

(45) Veröffentlichungstag der Patentschrifft:
17.01.90

(51) Int. Cl. ⁴: **A 61 K 9/50**

(21) Anmeldenummer: **85905803.4**

(22) Anmeldetag: **02.11.85**

(86) Internationale Anmeldenummer:
**PCT/EP 85/00585**

(87) Internationale Veröffentlichungsnummer:
**WO 86/02834 (22.05.86 Gazette 86/11)**

(54) PHARMAZEUTISCHE ZUBEREITUNGEN IN FORM VON INSTANTGRANULATEN ODER -TABLETTEN, SOWIE VERFAHREN ZU DEREN HERSTELLUNG.

(30) Priorität: **05.11.84 DE 3440288**

(43) Veröffentlichungstag der Anmeldung:
**19.08.87 Patentblatt 87/34**

(45) Bekanntmachung des Hinweises auf die Patenterteilung:
**17.01.90 Patentblatt 90/03**

(84) Bennante Vertragsstaaten:
**AT BE CH DE FR GB IT LI LU NL SE**

(56) Entgegenhaltungen:
**AT-C-368 880**
**FR-A-2 336 919**
**FR-A-2 552 308**

**Patent abstracts of Japan, section C, Band 8 nr.34 (C210) (1471), 15 Februar 1984, Tokyo JP**

(73) Patentinhaber: **Gergely, Gerhard, Dr.**
**Gartengasse 8**
**A-1050 Wien (AT)**

(72) Erfinder: **GERGELY, Thomas**
**Gartengasse 8**
**A-1050 Wien (AT)**
Erfinder: **GERGELY, Irmgard**
**Gartengasse 8**
**A-1050 Wien (AT)**

(74) Vertreter: **Büchel, Kurt F., Dr.**
**Patentanwalt Dr. Kurt F. Büchel Bergstrasse 297**
**FL-9495 Triesen (LI)**

EP 0 232 277 B1

2

**Beschreibung**

Die Erfindung betrifft eine pharmazeutische Zubereitung, die wenigstens einen pharmazeutischen Wirkstoff (unter Ausschluss von Ibuprofen, siehe EU-A-0 181 564), insbesondere einen unlöslichen oder langsam, bzw. schwer löslichen Wirkstoff enthält, und die wenigstens ein körniges Trägermaterial und eine das Trägermaterial umhüllende, andere Mischungsbestandteile enthaltende Schicht aufweist.

Bei der Herstellung pharmazeutischer Zubereitungen aus festen Wirkstoffen für die orale Applikation geht man meistens davon aus, die Wirkstoffe mit geschmacksverbessernden Füll- und gegebenenfalls Farb- und/oder Aromastoffen, diversen Hilfsstoffen, wie z. B. Schutzkolloiden, Sprengmitteln od. dgl. zu mischen, nötigenfalls zu granulieren, anschliessend auf gewünschte Korngrösse zu zerkleinern und sodann zu Tabletten zu verpressen, die dann gegebenenfalls noch dragiert werden. In diesen Tabletten liegt der Wirkstoff sogar bei bestmöglicher Mischung randomisiert vor.

In letzter Zeit beginnt sich allerdings mehr und mehr die Erkenntnis durchzusetzen, dass viele Arzneimittel zusammen mit einer grösseren Menge Wasser eingenommen werden sollen, insbesondere dann, wenn die zu verabreichenden Dosen grössere Mengen sind, wie z. B. 1 000 mg eines Wirkstoffes pro Gabe. Man versucht daher, die Zubereitungen in die Form von Instantpulvern oder -tabletten, oder von Brausepulvern oder -tabletten zu bringen, die vor der Einnahme in Wasser - ohne oder mit Entwicklung von Kohlensäure - gelöst oder suspendiert werden.

Die Resorption von Wirkstoffen durch den menschlichen Körper stellt ein Verteilungsproblem dar. Wenn Wirkstoffe, die in geringen Mengen vorliegen, optimal an den Körper herangebracht werden sollen, dann ist es zweckmässig, diese vorzuverdünnen oder aufzulösen, da damit die für die Resorption durch den Körper zur Verfügung stehende Oberfläche des Wirkstoffes vergrössert, bzw. besser ausgenützt wird. Beispielsweise würde Eisengluconat, wenn es nicht in Wasser aufgelöst wird, die Magenschleimhaut angreifen und zu Nebenerscheinungen führen.

Bei Antibiotika oder allen wasserunlöslichen Stoffen sollte z. B. die Substanz, um besser resorbiert zu werden, im Wasser in feinster Form suspendiert werden.

Bei der Herstellung von Instantpulvern oder -tabletten aus unlöslichen oder schwerlöslichen, gegebenenfalls bitter schmeckenden Wirkstoffen ergeben sich dabei die verschiedensten Probleme. Zum einen ist es durch die verschiedene Korngrösse der einzelnen Bestandteile der Mischung oft schwierig, sie exakt zu dosieren. Zum anderen zerfallen bei der Verwendung von leichtlöslichen Zuckern (zwecks Geschmacksverbesserung) Tabletten trotz des Einbaues von Sprengmitteln nur sehr langsam, weil die an der Oberfläche entstehende konzentrierte Zuckerlösung die Kapillaren des Sprengmittels verstopft und daher seine Wirkung zunichte macht oder zumindest sehr verlangsamt.

Die Erfindung hat sich daher zur Aufgabe gestellt, ein System zu schaffen, das die Herstellung von Instantgranulaten oder -tabletten so verbessert, dass Dosierungsprobleme der einzelnen Komponenten bei der Herstellung vermieden werden und die Granulate oder Tabletten in Wasser von Raumtemperatur innerhalb von 20 bis höchstens 40 Sekunden zerfallen, bzw. sich lösen. Dies wird durch die im folgenden kennzeichnenden Massnahmen erreicht.

Sie bestehen darin, daß eine wenigstens einen pharmazeutischen, wirkstoff, insbesondere einen unlöslichen oder schwer, bzw. langsam löslichen Wirkstoff (unter Ausschluss von Ibuprofen) enthaltende Zubereitung, die mindestens ein körniges Trägermaterial aus löslichem Kohlehydrat und eine das Trägermaterial umhüllende, andere Mischungsbestandteile enthaltende Schicht aufweist, dadurch gekennzeichnet ist das 5 bis 15, insbesondere 10 % des Wirkstoffes (auf Trägerkorn bezogen) - und gegebenenfalls ein Sprengmittel - direkt in die Oberfläche jedes Kornes eingebettet, oder 10 bis 50, insbesondere 20 bis 30 Gewichtsprozent (auf Trägerkorn bezogen) des Wirkstoffes - und gegebenenfalls ein Sprengmittel - an der Oberfläche einer das Trägerkorn umhüllenden Bindemittelschicht verankert sind.

Ferner ist diese Zubereitung dadurch gekennzeichnet, das der Wirkstoff eine schleimhautreizende Substanz aus der Gruppe der Profene - unter Ausschluss von Ibuprofen - ist, wobei die Profen-Partikel mit einem Überzug aus Fumarsäure und wenigstens einem Pseudokolloid, insbesondere Xanthan und/oder Maltodextrin, umhüllt sind.

In der Granulatform ist das Mittel dadurch gekennzeichnet, das es etwa gleich grosse Granulatkörner einer Brausemischung enthält, die vorzugsweise Zitronensäure und Calciumcarbonat aufweist, wobei das Calciumcarbonat die Zitronensäure unter Haftvermittlung durch eine Bindeschicht umhüllt, welche durch Anreaktion des Calciumcarbonates mit der oberflächennahen Schicht der Zitronensäurekristalle gebildet ist.

Die entsprechende Zubereitung für wasserempfindliche Wirkstoffe ist, dadurch gekennzeichnet, das der Wirkstoff, insbesondere Amoxicillintrihydrat, mit Hilfe einer aus Fett - insbesondere aus gehärtetem Rizinusöl - und einem Emulgator bestehenden Bindemittelschicht an dem Trägerkorn verankert ist.

Des weiteren ist die erfindungsgemäße dadurch gekennzeichnet, das der Wirkstoff mindestens ein Antibiotikum, insbesondere Ampicillin, Erythromycin und/oder Amoxicillin ist, dass der Wirkstoff, insbesondere Tryptophan, in hoher Dosierung, insbesondere von mindestens 0.5 g pro Gabe, vorliegt; ferner daß die Zubereitung dadurch gekennzeichnet, ist das sie einen in wässriger Suspension instabilen Wirkstoff, insbesondere Carbocistein, Acelastin und/oder ein Vitamin enthält.

Das Verfahren zur Herstellung einer pharmazeutischen Zubereitung nach einem der vorangehenden Ansprüche, zeichnet, sich dadurch aus das die Oberfläche jedes Trägerkornes unter Vakuum mit einem Lösungsmittel, insbesondere mit Wasser benetzt wird, worauf gegebenenfalls eine teilweise Antrocknung der Kornoberfläche erfolgt, und dass anschliessend der mikronisierte Wirkstoff eingetragen und unter Mischen auf der Kornoberfläche verankert wird, worauf das entstandene Granulat unter Vakuum fertig getrocknet wird.

Im Lösungsmittel können dabei ein oder mehrere der folgenden Substanzen gelöst oder suspendiert sein: ein niedriger Alkohol, ein oder mehrere Bindemittel, Schutzkolloide, Farbstoffe und/oder Aromen oder dergleichen Hilfs- oder Zusatzstoffe.

Nach einer andere bevorzugten Ausführungsform wird jedes Trägerkorn in einem beheizten Vakuummischer mit einer Schmelze aus Fett überzogen, die vorzugsweise einen Emulgator enthält, worauf - gegebenenfalls nach teilweiser Abkühlung - der Wirkstoff zugesetzt und unter Mischen an der Kornoberfläche verankert, und zum Schluß die Masse abgekühlt und ausgetragen wird.

Dabei liegen die Wirkstoffe nicht mehr randomisiert vor, sondern sind auf der Kornoberfläche kontrolliert verteilt und fixiert. Der Trägerstoff liegt vorzugsweise in einer Korngrösse von 0,2 bis 0,5 mm vor.

Die erfindungsgemässe pharmazeutische Zubereitung ermöglicht nun die Erzielung einer Suspension in Kontakt mit Wasser nahezu automatisch, indem nämlich die allfälligen Kolloide und der Wirkstoff in Wasser mit Hilfe des Sprengmittels von der Oberfläche des Kohlehydrat-Trägerkornes abgesprengt werden.

Speziell bei hohen Dosen ist das System interessant, weil eine Tablette, die beispielsweise ein halbes Gramm oder ein Gramm Antibiotikum enthält, zumindest 1 bis 2 Gramm schwer sein muss. Diese Tablette ist bereits schwer einzunehmen. Ausserdem würde sie beim Einnehmen zu Resorptionsproblemen führen, da im Magen/Verdauungstrakt eine zu hohe lokale Konzentration an Wirkstoff entsteht.

Bringt man das Produkt aber in Instant-Form ein, d. h. in Wasser suspendiert, dann entstehen keinerlei Verteilungsprobleme, es erfolgt auch eine raschere Resorption durch die grössere Oberfläche und vor allem ist das Produkt wesentlich angenehmer einzunehmen. Speziell Kinder und alte Leute ziehen ein Getränk einer Tablette vor.

Man hat zwar bereits vorgeschlagen (DE-A-3 434 774), auf beispielsweise Zitronensäurekristalle mikronisiertes Calciumcarbonat mit Hilfe einer Reaktions-Zwischenschicht aus Kalziumzitrat aufzubringen; doch sind dabei sowohl die Problematik (Herstellung einer natriumfreien oder -armen Brausemischung), wie auch die Massnahmen (Reaktionsprodukt aus Träger und Brause-"Wirkstoff" als Bindeschicht) grundsätzlich verschieden.

Auch das Beschichten von aus schwerlöslichen Kolloiden oder Pseudokolloiden bestehenden Trägersubstanzen mit in einem Lösungsmittel suspendierten Wirkstoffen ist schon vorgeschlagen worden (AT-B-368 880); dabei treten erst recht andere Probleme und Massnahmen auf, als wenn ein leichtlöslicher Träger, wie z. B. ein Zucker, zuerst mit einem Bindemittel beschichtet und anschliessend auf dem Bindemittel der Wirkstoff niedergeschlagen wird. Bei der genannten AT-B besteht das Produkt aus einem wasserunlöslichen, kolloidbildenden Trägerkorn mit angequollener Oberfläche, in die ein Wirkstoffpulver eingebettet ist. Es wird ohne Bindemittelschicht gearbeitet; das Trägerkorn wird mit dem Wirkstoff gemischt und dann ein Lösungsmittel zugefügt, das gegebenenfalls beide Partner anlöst. Die Kristallgrösse ist dabei schlecht steuerbar, weil auf das Trägerkorn und den Wirkstoff gleichzeitig Rücksicht genommen werden muss. Ausserdem löst sich das Trägerkorn schlechter auf.

Nun hat man zwar in der FR-A-2 336 919 bereits vorgeschlagen, Penicillin im Bindemittel zu suspendieren; die Suspension wird dann auf ein wasserlösliches Trägerkorn aufgebracht. Dabei ist nachteilig, dass das im Bindemittel eingebettete Penicillin im Kontakt mit Wasser nur langsam in Lösung geht. Bei der Herstellung ist eine grosse Lösungsmittelmenge erforderlich, was eine schlechte Energiebilanz ergibt. Ausserdem ist der zusätzliche Verfahrensschritt der getrennten Herstellung der Suspension erforderlich; die Methode eignet sich daher nur für unlösliche Wirkstoffe. Schliesslich ist auch die Kristallgrösse schlecht steuerbar.

Als Trägerstoff kommen erfindungsgemäss lösliche Kohlehydrate in Betracht, wie beispielsweise Kristallzucker, Sorbitol, Xylitol, geschmolzenes Mannitol, etc. Der Träger soll möglichst in einer gleichmässigen Grösse vorliegen. Seine Oberfläche soll regelmässig sein und darf keinen Staub enthalten.

Bisher wurden Instantgranulate unter anderem auf Basis von Pellets aufgebaut. Dies hat jedoch mehrere Nachteile: Pelletisieren ist ein unnötiger Schritt, der den Herstellvorgang verteuert und verlangsamt; er bringt mehr Bindemittel als erwünscht oder als nötig ein; Pellets sind - u.a. aus diesem Grund - schwerer löslich als Kristalle; Kristalle oder gemahlenes Schmelzgranulat haben eine unregelmässigere und damit grössere Oberfläche für die Verankerung von Wirkstoffen als die in aller Regel etwa kugelförmigen Pellets.

Auf der Oberfläche jedes Trägerkornes sind nun der Wirkstoff - und gegebenenfalls ein Sprengmittel - verankert, und zwar mit oder ohne Zuhilfenahme einer Bindemittelschicht. Eine solche Überzugsschicht auf jedem Trägerkorn enthält z. B. Dextrin, Polyvinylpyrrolidon oder dergleichen Bindemittel, und/oder Kolloide, wie Alginate, Xanthangummi, Gelatine, Maltodextrin od.dgl. und bewirkt übrigens unter anderem die Verlangsamung der Auflösung des Zuckerkornes;

anschliessend wird auf der noch feuchten Schicht mikronisierter Wirkstoff verankert. Arbeitet man nur mit Wasser (und gegebenenfalls Äthanol), dann gelingt es, etwa 5 bis 15, insbesondere etwa 10 Gewichtsprozent (auf Trägerkorn bezogen) des mikronisierten Wirkstoffes (und gegebenenfalls ein Sprengmittel) auf der Trägerkornoberfläche zu verankern. Will man 10 bis 50, insbesondere wie oft gewünscht etwa 20 bis 30 Gewichtsprozent verankern, bedient man sich der erwähnten Bindemittelschicht.

Die Schichtdicke des Bindemittels, die auf dem Träger erzeugt wird, hat eine Stärke von 4 bis 20 micron. Solche Schichten können aber mehrmals aufgetragen werden, so dass auch Schichtdicken, insbesondere bei gleichzeitiger Verankerung grösserer Wirkstoffmengen, von 50 bis 200 micron um den Carrier herum möglich sind.

Gleichzeitig oder anschliessend kann wie erwähnt auch ein Sprengmittel mit aufgebracht werden, das beim Kontakt mit Wasser die Schicht absprengt, so dass die Wirkstoffe, allenfalls begünstigt durch Schutzkolloide, suspendiert werden. Dabei geht dann gleichzeitig bzw. anschliessend der Zucker in Lösung, so dass am Ende des Vorganges nach 20 - 30 Sekunden und Rühren mit einem Löffel eine wohlschmeckende Suspension eines Wirkstoffes in Wasser entsteht. Als Sprengmittel kommen in an sich bekannter Weise z. B. native Stärke, wie Maisstärke; Mikrozellulose; oder auch quervernetztes Polyvinylpyrrolidon in Frage. Wichtig ist natürlich, dass die Bindemittelschicht - soferne überhaupt ein Bindemittel verwendet wird - möglichst dünn, wasserlöslich oder gut wassersuspendierbar ist, damit die Wirkung des Sprengmittels in Wasser nicht aufgehoben ist.

Die Struktur kann weiters noch Puffer zur Stabilitätserhöhung des Wirkstoffes, Farbstoffe zur Herstellung gefärbter Lösungen, Sülsstoffe, etc. enthalten. Weitere Begleitstoffe zu diesem Trägersystem sind Aromastoffe und Trocknungsmittel. Beispiele dafür sind verkapselte Aromen, die ebenfalls in der Grösse des Endproduktes, vorzugsweise 0,3 bis 0,5 mm, vorliegen müssen, damit beim Verfüllen der Mischungen keine Entmischung stattfindet und die Produktuniformität gewahrt bleibt. Auch können Natriumsulfat und/oder -karbonat in grobkörniger Form beigefügt werden, die beim Verpacken und beim Lagern das System trocken halten.

Das Verfahren zur Herstellung der erfindungsgemässen pharmazeutischen Zubereitung besteht im wesentlichen darin, dass zunächst im Vakuum die Luft an der Oberfläche des Trägers entfernt wird, um einen intensiven Kontakt zwischen der nachfolgenden Schicht und dem Träger herzustellen. Auf diesen hochevakuierten Träger wird sodann ein Lösungsmittel, vorzugsweise mit einem Bindemittel, beispielsweise Wasser mit einem Dextrin, Polyvinylpyrrolidon, etc. aufgebracht; dadurch wird der Zucker oberflächlich angelöst, so dass auf seiner Oberfläche ein Film entsteht, der aus einer konzentrierten Lösung des Trägers mit bereits eingebautem Bindemittel und/oder Kolloid besteht. In aller Regel muss vor, während und/oder nach dem Aufbringen der Oberflächenschicht die Trägermasse durch Mantel- oder Direktheizung aufgewärmt werden, damit das Lösungsmittel leichter verdampft und man zu besser klebrigen - weil höher viskosen - höheren Konzentrationen in der Oberflächenschicht kommt. Auf diese klebende Oberfläche wird sodann die Wirkstoffkomponente mit gleichzeitiger oder nachfolgender Sprengmittelzugabe aufgebracht.

Nachdem die Schicht durch entsprechende technische Massnahmen gleichmässig auf dem Träger verteilt ist, wird durch Anlegen von Vakuum getrocknet. Handelt es sich um feuchtigkeitsempfindliche Stoffe, so wird vorzugsweise zur Entfernung der Restfeuchtigkeit eine Zusatzheizung verwendet.

Erst anschliessend können die erwähnten Zusatzstoffe, wie Aromen und Trocknungsmittel, hinzugefügt werden, und es entsteht ein absolut gleichförmiges, leicht rieselfähiges und leicht verpackbares Produkt, das übrigens unter Verwendung an sich bekannter hydrophiler Gleitmittel natürlich auch zu Instant- bzw. gegebenenfalls Brausetabletten verpresst werden kann.

Das erfindungsgemässe System kann aber nicht nur mit Wasser und/oder Äthanol aufgebaut werden. Wenn Wirkstoffe auf den Träger aufgetragen werden sollen, die wasserempfindlich sind, kann man folgendermassen vorgehen:

Ein Gemisch aus Fett und einem Emulgator wird aufgeschmolzen und auf den - vorzugsweise in einer evakuierten Mischtrommel befindlichen - Träger aufgetragen, der soweit erwärmt sein muss, dass es nicht zur Verfestigung des Fettgemisches kommt. Dieses wird sich dann im Vakuum (durch Luftausschluss) gleichmässig verteilen und eine Schichte von Fett mit Emulgator an der Oberfläche des Trägers aufweisen. Auf diese Schichte kann nun der wasserempfindliche Wirkstoff - wie in vorhergehender Weise gegebenenfalls auch zusammen mit einem Sprengmittel - aufgebracht und verteilt werden, wobei beim langsamen Verfestigen des Fetts mit dem Emulgator durch Abkühlen und weiterfolgendem Rühren der Wirkstoff an dem Träger fixiert wird.

Die erfindungsgemässe Zubereitung ist auch in besonders vorteilhafter Weise dazu geeignet, ein Mischsystem mit Brausewirkung herzustellen. Will man beispielsweise ein Multivitamin-Mineral-Präparat herstellen, bei dem die Mineralien die Beständigkeit von Vitaminen stören, ist folgender Weg gangbar:

Man stellt ein Trägersystem her, das vermittels eines Brausezusatzes, vorzugsweise aus Calciumcarbonat und/oder Natriumcarbonat mit Zitronensäure, an der Oberfläche der Zuckerkristalle oder auch an der Oberfläche von z. B. Zitronensäurekristallen ein Trägerbrausesystem darstellt. In diesem System können diejenigen Mineralien untergebracht werden, die mit ihm kompatibel sind, also beispielsweise Eisensalze, Calciumsalze, Magnesium und dergleichen.

Dabei kann vorgesehen sein, dass die Brausemischung Zitronensäure und Calciumcarbonat aufweist, wobei das Calciumcarbonat die Zitronensäure unter Haftvermittlung durch eine Bindeschicht umhüllt, die durch Anreaktion des Calciumcarbonates mit der oberflächennahen Schicht der Zitronensäurekristalle gebildet ist.

Um die für diesen Vorgang empfindlichen Vitamine in das Systemeinzubringen, verfährt man wie oben geschilert bei den Zucker- und Sorbitolträgern, indem man die Vitamine vermittels Binde- und Sprengmittel auf einen Kohlehydratträger aufbringt.

Da beide Systeme in denselben Teilchendimensionen hergestellt werden können (vorzugsweise 0,2 bis 0,5 mm), können sie zusammengemischt werden und entmischen sich beim Abpacken in Sachets nicht.

Es ist auch bekannt, dass es Wirkstoffe gibt, die aus einer in Mund- und Rachenhöhle stark schleimhautreizenden Säure bestehen bzw. einen diesbezüglich stark reizenden Säurerest aufweisen. Derartige, im übrigen schwer- bis unlösliche Wirkstoffe sind die Profene (generischer Name), meist Propionsäure- oder auch Benzenderivate, von denen Abkömmlinge mit verschieden starker Reizwirkung bekannt sind. Unter der Bezeichnung Profen werden hier wie im folgenden alle schwer- bzw. unlöslichen Substanzen ähnlicher Struktur - unter Ausschluss von Ibuprofen - mit im vorbeschriebenen Sinne stark schleimhautreizenden Säureresten etc. verstanden.

Die Profene sind Wirkstoffe, die zur Rheuma- und Arthritisbekämpfung zunehmende Bedeutung gewonnen haben. Sie sind meist in Wasser unlöslich, haben aber einen sehr unangenehmen Geschmack. Insbesondere werden die Schleimhäute der Speiseröhre gereizt, offensichtlich insbesondere einerseits durch die Zusammensetzung, andererseits durch die Konsistenz. Ebenfalls literaturbekannt ist, dass die Profene gastrointestinales Bluten hervorrufen können, ähnlich wie die Acetylsalicylsäure, insbesondere wenn die Substanzen in höherer Konzentration, sei es in Form einer Tablette oder einer Kapsel, an die Magenwand geraten. Es ist daher ein therapeutischer Vorteil, wenn diese Substanzen bereits vor Einnahme in Wasser suspendiert werden, sodass sich lokale Überkonzentrationen im gastrointestinalen Trakt nicht mehr entwickeln können. Diese Suspendierung in Wasser ist aber aus geschmacklichen Gründen ohne zusätzliche Massnahmen nicht möglich, da Teile des Wirkstoffes im Mund und an der Speiseröhre hängenbleiben und dort zu kratzenden und reizenden Geschmackssensationen führen.

Der Erfindung liegt nun ausserdem die Aufgabe zugrunde, eine pharmazeutische Zubereitung der eingangs genannten Art, sowie ein Verfahren zu deren Herstellung anzugeben, welche den vorstehend beschriebenen negativen geschmacklichen Effekt beseitigen und die problemlose orale Einnahme von an sich schleimhautreizenden Arzneimitteln gewährleisten.

Diese Aufgabe wird dadurch gelöst, dass die Partikel der schleimhautreizenden Substanz mit einem Überzug aus Fumarsäure und wenigstens einem Pseudokolloid, wie z. B. Xanthan und/oder Maltodextrin umhüllt sind. Vorzugsweise werden dann die umhüllten Partikel ihrerseits erfindungsgemäss auf Trägerkörner aus Kohlehydrat aufgebracht.

Das erfindungsgemäss vorgeschlagene Verfahren zum Herstellen einer pharmazeutischen Zubereitung der erfindungsgemässen Art ist dadurch gekennzeichnet, dass die Profenpartikel in einer Vakuummischmaschine unter Vakuum mit dem Überzug aus dem wenigstens einen Pseudokolloid und Fumarsäure umhüllt und anschliessend vakuumgetrocknet werden.

Dabei kann insbesondere vorgesehen sein, dass zunächst das Profen und das Pseudokolloid bzw. die Pseudokolloide mit Wasser in der Vakuummischmaschine bei einem Druck von ca. 0,1 bar gemischt werden; und dass nach Antrocknen auf ca. 0,2 bar die Fumarsäure zugegeben wird, woraufhin das völlige Trocknen erfolgt.

Der Erfindung liegt die Erkenntnis zugrunde, dass der negative geschmackliche Effekt der bisherigen Profenpräparate dadurch beseitigt werden kann, dass man die Profenpartikel mit wenigstens einem Pseudokolloid und Fumarsäure umhüllt; dadurch wird bei gleichzeitiger Anwesenheit von Fumarsäure, die einen niedrigeren pH-Wert aufweist, der negative Geschmackeeffekt verhindert; die Pseudokolloide bewerkstelligen lediglich die Funktion der Verklammerung des wasserunlöslichen Profens mit der schwer löslichen Fumarsäure. Die Herstellung der erfindungsgemässen pharmazeutischen Zubereitung bzw. die Durchführung des erfindungsgemässen Verfahrens erfolgen dabei im übrigen zweckmässiger- und vorteilhafterweise mittels einer Vakuummischmaschine und mittels eines Verfahrens, wie es Gegenstand der DE-A-3 434 774.7 ist, auf die zur ergänzenden Erläuterung des Erfindungsgedankens insoweit in vollem Umfang Bezug genommen wird.

**Beispiel 1:**

80 Teile Griesszucker der Korngrösse 0,5 bis 0,2 mm werden mit 1,5 Teilen Natriumcyclamat in einen auf 80 Grad C vorgeheizten Vakuumischer eingesaugt. Eine Lösung bestehend aus 70 %-igem Äthylalkohol und 2 bis 3 Teilen Polyvinylpyrrolidon wird nach dem Evakuieren in den Vakuumischer eingesaugt und 3 Minuten schwingend gemischt, um die darin befindlichen Trägerkörner zu benetzen. Es werden dann 11 Teile Tryptophan eingesaugt, unter schwingendem Mischen während 5 Minuten verteilt und an der Oberfläche des Trägers aufgeklebt. Anschliessend wird vakuumgetrocknet bis zu einem Endwert von 15 mbar $10^{-8}$ Pa und sodann mit 0,6 Teilen Orangenaroma und 7 Teilen Zitronensäure, die sich beide in der Korngrösse von etwa 0,5 bis 0,3 mm an den umhüllten Träger angleichen sollen, gemischt. Diese Art der Zubereitung ist insbesondere für die bei Tryptophan meist erforderliche hohe Dosierung zweckmässig.

**Beispiel 2:**

Für ein Multivitamin-Mineral-Brausegranulat werden die beiden Phasen getrennt hergestellt. Herstellung der mineralhältigen Brausephase: In einen Vakuummischkessel mit einer Manteltemperatur von 80 Grad C werden 48 Teile kristallisierte Zitronensäure, 6 Teile Trinatriumzitrat, 2 Teile Vitamin C, alle mit der Korngrösse 0,1 bis 0,4 mm, als Carrier mit 0,6 Teilen Saccharin und den Mineralsalzen, wie z. B. 0,6 Teilen Eisengluconat, 0,01 Teilen Natriumfluorid und gewünschtenfalls Farbstoffen, in den Vakuumgranulator eingesaugt und unter Mischen auf 50 Grad C aufgeheizt. Sodann wird eine Lösung von 2 Teilen pulverisierter Zitronensäure in 0,7 Teilen Wasser hergestellt und mit 1 Teil Äthanol gemischt. Die aufgeheizte Masse wird evakuiert; anschliessend saugt man die Lösung ein und verteilt unter schwingendem Mischen während 5 Minuten auf dem Carrier. Sodann bringt man 6 Teile gefälltes Calciumcarbonat, 2 Teile Magnesiumoxyd und 4 Teile Natriumcarbonat ein, und verteilt unter Mischen während 2 bis 5 Minuten die Substanzen auf dem Carrier; anschliessend wird die Masse mittels Vakuum 20 Minuten lang getrocknet. Abschliessend werden 9 Teile Natriumcarbonat und 2 Teile Orangenaroma zugemischt, und die Masse wird über eine Siebvorrichtung ausgetragen.

Die beiden Phasen werden anschliessend in der gewünschten Relation zusammengemischt.

**Beispiel 3:**

80 bis 90 Teile Kristallzucker der Korngrösse 0,6 bis 0,2 mm werden in einem Vakuummischer mit Mantelheizung auf 80 Gradliaufgeheizt. Inzwischen wird eine Schmelze aus 3 Teilen gehärtetem Rhizinusöl und 0,1 bis 0,2 Teilen eines Emulgators (z. B. "Emulgin B1" der Firma Henkel, ein Cetylstearylalkohol mit einem Erstarrungsbereich zwischen 32 und 37 Grad C) hergestellt. Die Schmelze wird in die Mischtrommel eingebracht und die Manteltemperatur auf 60 Grad C abgekühlt. Inzwischen verteilt sich unter schwingendem Mischen die Schmelze auf der Oberfläche der Zuckerkristallkörner. Anschliessend gibt man unter weiterem schwingendem Mischen 7 Teile Amoxicillintrihydrat zu, schaltet die Kühlung ein und kühlt die Manteltemperatur auf 30 Grad ab, wobei die Masse bis auf 65 Grad C abkühlt und die Schmelze erstarrt. Unter auflockerndem Mischen wird sodann 1 Teil Kokosaroma zugemischt und die Masse anschliessend durch eine Siebmaschine ausgetragen.

**Beispiel 4:**

Es kann auch ein in einer Matrix eingebetteter Wirkstoff, im vorliegenden Fall Acelastinbase, erfindungsgemäss in einem Getränk optimal suspendiert werden.

**Beispiel 5:**

85 Teile Instant Sorbitol (sprühgetrocknetes Sorbit einer Korngrösse von 0,4 bis 0,1 mm) werden in einen auf 70 Grad C vorgeheizten Vakuummischer eingesaugt. Eine Lösung von 4 Teilen Karion flüssig, in der der Farbstoff gelöst ist, wird in den Vakuummischer eingesaugt und 5 Minuten lang verteilt, um den darin befindlichen Carrier zu benetzen. Es werden dann 5 Teile Ampicillin anhydr. in den Mischer eingebracht und unter schwingendem Rühren an der Oberfläche des Trägers fixiert. Anschliessend werden auf die noch feuchte Oberfläche 1,5 Teile Karayagummi, 1,5 Teile Carboxymethylcellulose und 1 Teil Maisstärke aufgebracht. Sodann wird unter langsamem Rühren unter Vakuum auf einen Endwert von 15 mbar getrocknet. Zum Schluss fügt man der Masse 0,1 Teile Bananenaroma und 2 Teile Natriumsulfat zu.

**Beispiel 6:**

80 Teile geschmolzenes Mannitol, das anschliessend auf 0,4 bis 0,1 mm Korngrösse gemahlen wurde, werden in einem Vakuummischer mit Mantelheizung auf 50 Grad C aufgeheizt. Eine Lösung, bestehend aus 3 Teilen Zucker, 3 Teilen Wasser und 0,3 Teilen Maltodextrin, wird nach dem Evakuieren in den Vakuummischer eingesaugt und auf dem Träger durch Rühren 3 Minuten lang verteilt. Es werden sodann 7 Teile Erythromycinsuccinat in den Vakuummischer eingesaugt und unter schwingendem Mischen 5 Minuten lang an der Carrieroberfläche verteilt. Der Wirkstoff ist so unlöslich, dass er aus der Bindemittelschicht beim Kontakt mit Wasser von selbst abspringt; daher kann er ohne Sprengmittel verarbeitet werden. Anschliessend werden 1,5 Teile Maltodextrin und 1,5 Teile eines Alginates auf die Trägeroberfläche aufgebracht; sodann wird unter langsamem Rühren bis zu einem Endwert von 20 mbar Vakuum getrocknet. Unter langsamem Mischen werden sodann 0,1 Teile Orangenaroma und 2 Teile Natriumsulfat zugemischt.

**Beispiel 7:**

In einem beheizbaren Vakuummischer werden 40 Teile Kristallzucker, 2 Teile Natriumphosphat, 6 Teile Natriumcitrat in gleicher Korngrösse und die gewünschte Menge Süssstoffe eingesaugt und unter Mischen auf 50 Grad C aufgeheizt. Vor dem Einsaugen der Lösung, bestehend aus: 0,3 Teilen Dextrin, 3 Teilen Zucker, 1,5 Teilen Alkohol und 2 Teilen Wasser, wird evakuiert, die Lösung sodann auf dem Carrier durch Mischen verteilt und sodann an der benetzten Carrier-Oberfläche 14 Teile Carbocistein angeklebt. Anschliessend werden 3 Teile Dextrin, 3 Teile Alginat, 0,6 Teile Polyvinylpyrrolidon sowie 3 Teile Maisstärke eingesaugt und die Oberfläche der Kristalle damit abgedeckt. Sodann wird das Produkt vakuumgetrocknet und abschliessend werden übliche Trockenmittel, Aromen, Stabilisierungsmittel und die restlichen Begleitstoffe zugemischt.

**Beispiel 8:**

200 Teile Suprofen werden mit einer Lösung von 10 Teilen Xanthan und 10 Teilen Maltodextrin in 50 Teilen Wasser behandelt; vor dem völligen Trocknen in Vakuum bei ca. 200 mbar werden 20 Teile Fumarsäure zugesetzt. Es entsteht nach dem völligen Trocknen ein Granulat, das auf eine Korngrösse von ca. 0,1 mm oder darunter gemahlen wird.

Diese Teilchen können nun nach der erfindungsgemässen Instant-Technologie auf Kristallzucker aufgebracht werden, wobei vorzugsweise die 10 bis 15-fache Menge Zucker angewendet wird und die doppelte Menge Zitronensäure hinzugefügt werden kann.

Stellt man ein derartiges Gemisch auf eine Einzeldosis von 200 mg Suprofen her, dann entsteht bei geeigneter Aromatisierung ein angenehmes Getränk, das keinerlei Sensationen auf die Schleimhäute mehr ausübt.

**Beispiel 9:**

Dasselbe System, wie vorher geschildert, kann verbessert werden, indem man eine Brausemischung von Zitronensäure und Calciumcarbonat beifügt. Man lässt 50 Teile Zitronensäure mit 50 Teilen Calciumcarbonat im Vakuum reagieren, indem man 5 Teile 50 %-igen Äthylalkohols hinzufügt. Dieser Brauseteil wird getrocknet und dem Beispiel 8 in 5-facher Menge zum Suprofen hinzugefügt.

Ein so hergestelltes Produkt zeigt einen selbstsuspendierenden Effekt einer langsamen Brausemischung, wobei offensichtlich die vorhandenen Kalziumionen einen weiteren verbessernden Effekt auf den Geschmack des Suprofen ausüben.

**Beispiel 10:**

3 000 Teile Feinkristallzucker werden mit einer Lösung von 60 Teilen Wasser und 40 Teilen Maltodextrin bei 60 Grad C versetzt; die Lösung wird im Vakuummischkessel gleichmässig auf dem Kristallzucker verteilt.

Sodann werden 200 Teile Naproxen auf der Oberfläche des Zuckers verteilt und 20 Teile Fumarsäure aufgebracht. Nach deren völligem Verteilen wird auf 50 mbar getrocknet, und man bringt feinpulverisierte Zitronensäure der Korngrösse unter 100 micron ein, mit der man die Oberfläche des Trägers abdeckt. Anschliessend trocknet man mittels Vakuum auf 10 mbar.

Nach Zufügen gewünschter Mengen von Süssstoffen und Aromen entsteht ein gleichmässiges Granulat, das auf eine Einzeldosis von 200 bis 400 mg Naproxen in Einzelbeuteln verpackt werden kann.

**Patentansprüche**

1. Wenigstens einen pharmazeutischen Wirkstoff, insbesondere einen unlöslichen oder schwer, bzw. langsam löslichen Wirkstoff (unter Ausschluss von Ibuprofen) enthaltende Zubereitung, die wenigstens ein körniges Trägermaterial aus löslichem Kohlehydrat und eine das Trägermaterial umhüllende, andere Mischungsbestandteile enthaltende Schicht aufweist, dadurch gekennzeichnet, dass 5 bis 15, insbesondere 10 % des Wirkstoffes (auf Trägerkorn bezogen) - und gegebenenfalls ein Sprengmittel - direkt in die Oberfläche jedes Kornes eingebettet, oder 10 bis 50, insbesondere 20 bis 30 Gewichtsprozent (auf Trägerkorn bezogen) des Wirkstoffes - und gegebenenfalls ein Sprengmittel - an der Oberfläche einer das Trägerkorn umhüllenden Bindemittelschicht verankert sind.

2. Zubereitung nach Anspruch 1, dadurch gekennzeichnet, dass der Wirkstoff eine schleimhautreizende Substanz aus der Gruppe der Profene - unter Ausschluss von Ibuprofen - ist, wobei die Profen-Partikel mit einem Überzug aus Fumarsäure und wenigstens einem Pseudokolloid, insbesondere Xanthan und/oder Maltodextrin, umhüllt sind.

3. Zubereitung nach Anspruch 1 oder 2, in Granulatform, dadurch gekennzeichnet, dass sie etwa gleich grosse Granulatkörner einer Brausemischung enthält, die vorzugsweise Zitronensäure und Calciumcarbonat aufweist, wobei das Calciumcarbonat die Zitronensäure unter Haftvermittlung durch eine Bindeschicht umhüllt, die durch Anreaktion des Calciumcarbonates mit der oberflächennahen Schicht der Zitronensäurekristalle gebildet ist.

4. Zubereitung nach Anspruch 1 oder 2, für wasserempfindliche Wirkstoffe, dadurch gekennzeichnet, dass der Wirkstoff, insbesondere Amoxicillintrihydrat, mit Hilfe einer aus Fett - insbesondere aus gehärtetem Rizinusöl - und einem Emulgator bestehenden Bindemittelschicht an dem Trägerkorn verankert ist.

5. Zubereitung nach Anspruch 1 oder 2, dadurch gekennzeichnet, dass der Wirkstoff mindestens

## 13

ein Antibiotikum, insbesondere Ampicillin, Erythromycin und/oder Amoxicillin ist.

6. Zubereitung nach Anspruch 1 oder 2, dadurch gekennzeichnet, dass der Wirkstoff, insbesondere Tryptophan, in hoher Dosierung, insbesondere von mindestens 0.5 g pro Gabe, vorliegt.

7. Zubereitung nach Anspruch 1 oder 2, dadurch gekennzeichnet, dass sie einen in wässriger Suspension instabilen Wirkstoff, insbesondere Carbocistein, Acelastin und/oder ein Vitamin enthält.

8. Verfahren zur Herstellung einer pharmazeutischen Zubereitung nach einem der vorangehenden Ansprüche, dadurch gekennzeichnet, dass die Oberfläche jedes Trägerkornes unter Vakuum mit einem Lösungsmittel, insbesondere mit Wasser benetzt wird, worauf gegebenenfalls eine teilweise Antrocknung der Kornoberfläche erfolgt, und dass anschliessend der mikronisierte Wirkstoff eingetragen und unter Mischen auf der Kornoberfläche verankert wird, worauf das entstandene Granulat unter Vakuum fertig getrocknet wird.

9. Verfahren nach Anspruch 8, dadurch gekennzeichnet, dass in dem Lösungsmittel ein oder mehrere der folgenden Substanzen gelöst oder suspendiert sind: ein niedriger Alkohol, ein oder mehrere Bindemittel, Schutzkolloide, Farbstoffe und/oder Aromen oder dergleichen Hilfs- oder Zusatzstoffe.

10. Verfahren zur Herstellung einer pharmazeutischen Zubereitung nach Anspruch 4, dadurch gekennzeichnet, dass jedes Trägerkorn in einem beheizten Vakuummischer mit einer Schmelze aus Fett überzogen wird, die vorzugsweise einen Emulgator enthält, worauf - gegebenenfalls nach teilweiser Abkühlung - der Wirkstoff zugesetzt und unter Mischen an der Kornoberfläche verankert wird, und dass schliesslich die Masse abgekühlt und ausgetragen wird.

## Claims

1. A preparation containing at least one pharmaceutical active substance, especially an insoluble or sparingly soluble or slowly soluble active substance (with the exception of ibuprofene) that has at least one granular carrier material and a layer that contains other mixture components covering the carrier material, wherein every carrier grain consists of a crystal or of a homogeneous melted granulate of at least on - preferably freely or quickly soluble - carbohydrate, and wherein 5 to 15 %, especially 10 % of the active substance (relating to the carrier grain) - and eventually a disintegrant - is embedded directly in the surface of every grain, or 10 to 50 %, especially about 20 to 30 % by weight (relating to the carrier grain) of the active substance - and eventually a disintegrant - are anchored with the aid of a binding

## 14

agent layer to the surface of every grain.

2. Preparation as claimed in claim 1, wherein the active substance is a substance from the group of the profenes - excluding ibuprofene - that irritates mucous membranes, the profene particles being covered with a coating of fumaric acid and at least one pseudocolloid, especially xanthane and/or maltodextrine.

3. Preparation as claimed in claim 1 or 2, wherein said preparation contains an effervescent mixture that preferably contains citric acid and calcium carbonate, in which case the calcium carbonate covers the citric acid, the adhesion being provided by the binding layer that is formed by incipient reaction of the calcium carbonate with the layer of citric acid crystals near the surface.

4. Preparation as claimed in claim 1 or 2 for active substances sensitive to water, wherein the active substance, especially amoxicillin trihydrate, is anchored to the carrier grain with the aid of a binding agent layer consisting of fat - especially of hardened castor oil and an emulsifier.

5. Preparation as claimed in claim 1 or 2, wherein the active substance is at least an antibiotic, especially ampicillin, erythromycin and/or amoxicillin.

6. Preparation as claimed in claim 1 or 2, wherein the active substance, especially tryptophane, is present in a high dosage, especially of at least 0.5 g per dose.

7. Preparation as claimed in claim 1 or 2, wherein the active substance, especially carbocistein, acelastine and/or a vitamin, is unstable in aqueous suspension.

8. Process for the manufacture of a pharmaceutical preparation according to any one of the preceding claims, wherein the surface of every carrier grain - preferably in a vacuum mixing drum - is wetted with a solvent, especially water, whereupon partial drying of the grain surface eventually occurs, and wherein thereafter the micronized active substance is introduced and while mixed anchored to the grain surface, whereupon the obtained granulate is finishdried.

9. Process as claimed in claim 8, wherein one or more of the following substance is dissolved or suspended in the solvent: a low alcohol, one or more binding agents, protection colloids, coloring substances and/or aromatics or similar inactive ingredients or additives.

10. Process for manufacturing a pharmaceutical preparation according to claim 4, wherein every carrier grain is coated in a heated vacuum mixer with a melt of fat that preferably contains an emulsifier, whereupon - eventually after partial cooling down - the active substance is added and while being mixed anchored to the grain surface, and wherein finally the mass is cooled down and discharge.

## Revendications

1. Préparation contenant au moins une matière active pharmaceutique, en particulier une matière active (à l'exception de l'ibuprofène) insoluble, ou difficilement ou lentement soluble, laquelle préparation présente une matière support granulaire en hydrate de carbone soluble et une couche contenant d'autres ingrédients de mélange, enveloppant la matière support, caractérisée en ce que 5 à 15, en particulier environ 1 0 % de la matière active (par rapport aux granules supports) - et éventuellement un désagrégeant - sont directement insérés dans la surface de chaque grain, ou 10 à 50, en particulier environ 20 à 30 % en poids (par rapport aux granules supports) de la matière active - et éventuellement un désagrégeant - sont ancrés à la surface d'une couche de liant enveloppant les granules supports.

2. Préparation selon la revendication 1, caractérisée en ce que la matière active est une substance irritant les muqueuses, du groupe des profènes - à l'exception de l'ibuprofène - les particules de profène étant enveloppées d'un enrobage d'acide fumarique et d'au moins un pseudocolloïde, en particulier le xanthane et/ou la maltodextrine.

3. Préparation selon la revendication 1 ou 2, sous forme de granulés, caractérisée en ce qu'elle contient des grains de granulé de taille sensiblement égale d'un mélange effervescent qui présente de préférence de l'acide citrique et du carbonate de calcium, le carbonate de calcium enveloppant l'acide citrique par adhésion au moyen d'une couche de liant qui est formée par réaction du carbonate de calcium avec la couche voisine de la surface des cristaux d'acide citrique.

4. Préparation selon la revendication 1 ou 2 pour des matières actives sensibles à l'eau, caractérisée en ce que la matière active, en particulier le trihydrate d'amoxicilline, est ancrée sur les granules supports à l'aide d'une couche de liant constituée de graisse en particulier d'huile de ricin durcie - et d'un émulsifiant.

5. Préparation selon la revendication 1 ou 2, caractérisée en ce que la matière active au moins un antibiotique, en particulier l'ampicilline, l'érythromycine et/ou l'amoxicilline.

6. Préparation selon la revendication 1 ou 2, caractérisée en ce que la matière active, en particulier le tryptophane; est présente en une dose élevée, en particulier d'au moins 0,5 g par unité à administrer.

7. Préparation selon la revendication 1 ou 2, caractérisée en ce qu'elle contient une matière instable en suspension aqueuse; en particulier de la carbocistéine, de l'acélastine et/ou une vitamine.

8. Procédé pour la fabrication d'une préparation pharmaceutique selon l'une des revendications précédentes, caractérisé en ce que la surface de chaque grain support est humidifiée sous vide au moyen d'un solvant, en particulier l'eau, à la suite de quoi a lieu éventuellement un séchage partiel de la surface du grain, et en ce qu'ensuite la matière active micronisée est introduite et ancrée en mélangeant sur la du grain, à la suite de quoi la préparation des granulés résultants est terminée par séchage sous vide.

9. Procédé selon la revendication 8, caractérisé en ce que dans le solvant une ou plusieurs des substances suivantes sont dissoutes ou mises en suspension: un alcool inférieur, un ou plusieurs liants, des colloïdes protecteurs, des colorants et/ou des arômes, ou des adjuvants ou additifs analogues.

10. Procédé pour la fabrication d'une préparation pharmaceutique selon la revendication 4, caractérisé en ce que chaque grain support est enrobé d'une masse fondue de graisse, dans un mélangeur sous vide chauffé, laquelle masse fondue contient de préférence un émulsifiant, à la suite de quoi - éventuellement après refroidissement partiel - la matière active est ajoutée et ancrée à la surface des grains en mélangeant, et en ce que finalement la masse est refroidie et déchargée.